(19)

Europäisches
Patentamt
European
Patent Office
Office européen
des brevets

(11) **EP 3 132 790 A1**

(12) **EUROPEAN PATENT APPLICATION**
published in accordance with Art. 153(4) EPC

(43) Date of publication:
**22.02.2017 Bulletin 2017/08**

(21) Application number: **15779307.6**

(22) Date of filing: **16.04.2015**

(51) Int Cl.:
*A61K 9/16* (2006.01)    *A61K 38/22* (2006.01)
*A61K 47/34* (2017.01)    *A61P 3/10* (2006.01)

(86) International application number:
**PCT/CN2015/076688**

(87) International publication number:
**WO 2015/158270 (22.10.2015 Gazette 2015/42)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA**

(30) Priority: **16.04.2014   CN 201410153089
16.04.2014   CN 201410153581**

(71) Applicant: **Shandong Luye Pharmaceutical Co.,
Ltd.
Shandong 264670 (CN)**

(72) Inventors:
• **WANG, Tao**
**Yantai**
**Shandong 264000 (CN)**

• **WANG, Qilin**
**Yantai**
**Shandong 264003 (CN)**
• **XU, Qianqian**
**Yantai**
**Shandong 264000 (CN)**
• **SUN, Lifang**
**Yantai**
**Shandong 264003 (CN)**
• **MENG, Ying**
**Yantai**
**Shandong 264003 (CN)**
• **LI, Ju**
**Yantai**
**Shandong 264003 (CN)**

(74) Representative: **Rössler, Matthias**
**KNH Patentanwälte Kahlhöfer Neumann**
**Rößler Heine PartG mbB**
**Postfach 10 33 63**
**40024 Düsseldorf (DE)**

(54) **EXENATIDE-CONTAINING COMPOSITION AND PREPARATION METHOD THEREFOR**

(57)    The present invention relates to an exenatide-containing composition, and the composition is in the form of microspheres. The exenatide-containing microspheres use exenatide acetate and a copolymer of lactide and glycolide (PLGA) as raw materials, and the content of acetic acid in the prepared exenatide-containing composition is lower than 0.01%. In a manufacturing process of the microspheres, an appropriate amount of cleaning solution is used, an appropriate re-drying temperature is selected during the freeze-dried process, so as to obtain a composition of exenatide-containing microspheres with good characteristics meeting the quality standard, thereby iproving the stability and extending the shelf lives of the microspheres. In the manufacturing process of the microspheres, an appropriate outer aqueous phase is used to obtain microspheres with uniform particle size and minimum residue of the polymers, so as to provide a composition with better quality for clinical use.

**EP 3 132 790 A1**

Description

CROSS REFERECE TO RELATED APPLICATIONS

TECHNICAL FIELD

The present invention relates to pharmaceuticals, more particularly, to an exenatide-containing composition, which is in the form of microspheres. The present invention also relates to methods for preparing the same.

BACKGROUND

Exenatide is the first synthetic incretin analogue in the glucagon-like peptide-1 (GLP-1) family. It can be released from intestine into the circulatory system and leads to enhancement of glucose-dependent insulin secretion by mimicking the effects of GLP-1 secretion under normal physiological conditions in human. Exenatide shares partial amino acid sequence identity with human GLP-1. Several *in vivo* studies have shown that exenatide could bind to and activate known human GLP-1 receptors to promote, via cAMP and/or other intracellular signal transduction pathways, glucose-dependent synthesis of insulin and secretion of insulin from the pancreatic beta cells. Under the condition of elevated blood glucose concentration, exenatide can promote the release of insulin from pancreatic beta cells and reduce the levels of fasting blood glucose and postprandial blood glucose in type 2 diabetic patients, thereby improving glucose control.

The main exenatide products on the market are Exenatide injections, which can only be used for subcutaneous injection. The initial dose recommended is 5μg twice a day for one month. Then, the dose may be increased to 10μg based on the clinical responses. These pharmaceutical preparations are usually given by injection twice a day, and mainly eliminated by kidney. The average time to reach peak concentration is 2.1h, and the average elimination half-life is 2.4h. These injections require frequent administrations and cannot continuously activate the GLP-1 receptors. The compliance of the diabetics who need long term administration is poor.

Phase separation has already been used as a method for preparing exenatide-containing microspheres in the prior art. Phase separation involves many steps and requires high-level equipment and complex process. The microsphere products prepared by phase separation need to be further processed through several steps to meet pharmaceutical products related quality standard requirements, such as sterile requirement and solvent residue requirement. To overcome the aforementioned deficiencies, several institutes have used solvent evaporation technique as a preparation method for exenatide-containing microspheres. This technique involves fewer steps, needs lower-level equipment, and needs fewer steps to process the microspheres, as compared with phase separation method. The ingredient of the approved exenatide products is exenatide acetate. The standard requirement is: 3% ≤ acetic acid content ≤ 12%. The current processes for manufacturing exenatide-containing microspheres do not have any control of the acetic acid contents. Studies have shown that the contents of acetic acid in the exenatide-containing microspheres affect the bioavailabilities of the final products during storage, and that the bioavailabilities of exenatide in th microspheres decrease during storage when the content of acetic acid is above 0.01%.

SUMMARY

To solve the technical defects existing in the prior art, inventors of the present invention performed extensive research to provide exenatide-containing compositions in the form of microspheres. The exenatide-containing microspheres comprise exenatide acetate (approved product, 3% ≤the content of acetic acid ≤12%) and copolymers of lactide and glycolide (PLGA) as the starting materials. The contents of acetic acid in the microspheres thus prepared are below 0.01%.

The present invention provides exenatide-containing compositions. The compositions are in the form of microspheres. The exenatide-containing microspheres comprise exenatide acetate and copolymers of lactide and glycolide (PLGA) as starting materials. The contents of acetic acid in the prepared compositions are below 0.01%.

In a composition of the present invention, the content of exenatide is 2-10% by weight, preferably 3-8%, and more preferably 4-6%. The content of the copolymer of lactide and glycolide is 90-98% by weight, preferably 92-97%, and more preferably 94-96%.

A copolymer of lactide and glycolide is also referred to as poly (lactide-co-glycolide), abbreviated as PLGA. A PLGA for use in embodiments of the present invention has a molecular weight of 6,000-45,000 Dalton, preferably 10,000-30,000 Dalton, and more preferably 10,000-25,000 Dalton. As used herein, the term "molecular weight" refers to "weight average molecular weight," which is referred to herein as "molecular weight." The molar ratio of lactide to glycolide in said PLGA is from 90:10 to 10:90, preferably from 75:25 to 25:75, more preferably from 60:40 to 40:60, and especially 50:50. The intrinsic viscosity of PLGA is 0.10-0.40 dL/g, preferably in the range of 0.10-0.35 dL/g, and optimally in the range of 0.10-0.30dL/g. A method for measuring the intrinsic viscosity (also referred to as "inherent viscosity") of PLGA is as follows: prepare an about 0.5% (w/v) solution of PLGA in chloroform, and determine the intrinsic viscosity of PLGA at 30°C using a Cannon-Fenske glass capillary viscometer.

For convenience of description, the molar ratio of lactide to glycolide, the intrinsic viscosity and molecular weight of PLGA are shown hereinafter in brackets following each PLGA. For example, "PLGA (50/50, 0.20, 16,000)" represents poly (lactide-co-glycolide) with a molar ratio of lactide to glycolide of 50:50, an intrinsic viscosity of 0.20 dL/g, and a molecular weight of 16,000 Dalton.

Compositions in accordance with embodiments of the present invention may be prepared by an emulsion-solvent evaporation process. Therefore, embodiments of the present invention also provide methods for preparing exenatide-containing compositions.

The microspheres in accordance with embodiments of the present invention are prepared as follows: PLGA is dissolved in an organic solvent to form an organic solution; exenatide acetate is added into the PLGA organic solution to obtain a primary emulsion. Then, the primary emulsion is added into an outer aqueous phase and emulsified to obtain an emulsion. Then, the emulsion is solidified and volatilized to remove the organic solvent. The residue was washed and freeze-dried to obtain the freeze-dried microspheres.

The organic solvents can dissolve PLGA. These solvents may include poly (lactide-co-glycolide) (PLGA), dichloromethane, acetone, acetonitrile, and the like that can dissolve PLGA, preferably dichloromethane.

In the manufacturing process of a composition of the present invention, a cleaning solution in an appropriate proportion may be used, and a specific re-drying temperature may be set to dry the freeze-dried microspheres. The cleaning solution is selected from distilled water or an alkaline buffer solution, preferably distilled water. The weight ratio of the microspheres to the cleaning solution is 1:250 or above, preferably 1:300 or above. The specific re-drying temperature of freeze-dried microspheres is set at lower than the glass-transition temperature of the mixture of exenatide acetate and PLGA. By the aforesaid adjustment, a composition of exenatide-containing microspheres with good characteristic could be obtained, which contains a very low amount of acetic acid and meets the quality standard.

In the manufacturing process of a composition of the present invention, the concentration and pH value of outer aqueous phase may be adjusted. The outer aqueous phase is a polyvinyl alcohol solution, and the average molecular weight of the polyvinyl alcohol is 13,000-23,000. In the polyvinyl alcohol solution, the content of polyvinyl alcohol is 0.5-1.5% w/v, preferably 1% w/v. The PH value of the polyvinyl alcohol solution is $5.6\pm1.0$, preferably $5.6\pm0.5$, and more preferably $5.6\pm0.2$. Through these adjustments, the content of polyvinyl alcohol residue in a composition of the present invention should not be more than 0.008% (w/w %).

Specifically, the microspheres in accordance with embodiments of the present invention may be prepared by the solvent evaporation process as follows: A certain amount of exenatide or a salt or an analogue thereof is weighed and dissolved in distilled water to form an aqueous solution. Known amounts of excipients are weighed and dissolved in the aqueous solution. The excipients may include a cosolvent, a protein protectant, a surfactant, and/or a pore forming agent, etc. A certain amount of PLGA is weighed and dissolved in an organic solvent in which PLGA is soluble. The organic solvent may be selected from dichloromethane, acetone, acetonitrile, etc., preferably dichloromethane. The PLGA organic solution is then mixed with the aqueous solution containing exenatide and excipients to form a primary emulsion. The primary emulsion containing exenatide or a salt or an analogue thereof is added into an outer aqueous phase (e.g., a polyvinyl alcohol solution) and emulsified to obtain an emulsion. Then, the emulsion is solidified and volatilized to remove the organic solvent. The residue was washed and freeze-dried to obtain the microspheres.

In the aforesaid manufacturing process, exenatide acetate need not be pre-prepared to form an aqueous solution. Exenatide acetate may be mixed directly with the PLGA organic solution to form a primary emulsion.

In a manufacturing process of the microspheres according to embodiments of the present invention, the outer aqueous phase is a polyvinyl alcohol solution. In the polyvinyl alcohol solution, the content of polyvinyl alcohol is 0.5-1.5 w/v %, preferably 1 w/v%. The pH value of the solution is $5.6\pm1.0$, preferably $5.6\pm0.5$, and more preferably $5.6\pm0.2$.

The pH value of the outer aqueous phase plays a key role for the characteristics of the microspheres of the present invention. In accordance with embodiments of the present invention, the pH value of an outer aqueous phase may be adjusted by an acidic aqueous solution, a basic aqueous solution, or a buffer solution. The oil phase (primary emulsion) containing exenatide or a salt or an analogue thereof according to embodiments of the present invention is emulsified to obtain an emulsion that is solidified in an outer aqueous phase having a specific pH value. This provides a basis for the microspheres with good characteristics.

The pH value of the outer aqueous phase can be kept stable within a certain range by adjusting with an acid solution, a base solution, or a buffer. The acid may be a water-soluble acid, such as hydrochloric acid, sulfuric acid, acetic acid and amino acid, among which 0.1mol/L hydrochloric acid aqueous solution is commonly used. The base may be a water-soluble base, such as sodium hydroxide, potassium hydroxide, sodium bicarbonate and sodium sulfite, among which 0.1mol/L sodium hydroxide aqueous solution is commonly used. The buffer may be selected from citrate buffer, phosphate buffer, acetate buffer, and phthalate buffer, among which phthalate buffer (pH=5.6) is commonly used.

The aforementioned acid solution, base solution and buffer may be prepared in accordance with the appendix of Chinese Pharmacopeia (second part, 2010 Edition). For example, 0.1mol/L hydrochloric acid solution and 0.1mol/L sodium hydroxide solution may be prepared in accordance with the preparation method for titration solutions recorded in appendix XV F of Chinese Pharmacopeia, and phthalate buffer (pH=5.6) may be prepared in accordance with the preparation method for buffer solutions recorded in appendix XV D of Chinese Pharmacopeia.

In the manufacturing process of the microspheres described in the present invention, the weight/volume % of polyvinyl alcohol (g/100ml) in the outer aqueous phase plays a key role in the characteristics of the microspheres. In accordance with embodiments of the present invention, polyvinyl alcohol is 0.5-1.5 w/v %, preferably 1 w/v %. A composition of exenatide-containing microspheres with good characteristics may be obtained by solidifying the microspheres in the outer aqueous phase having such a selected concentration.

Polyvinyl alcohol used in the present invention may have an average molecular weight of 13,000-23,000 Dalton. A solution of such polyvinyl alcohol plays an important role in the characteristics of the microspheres thus prepared. Exenatide-containing microspheres with good characteristics may be obtained by solidifying the microspheres in an outer aqueous phase comprising polyvinyl alcohol which has such a selected average molecular weight.

In the manufacturing process of the present invention for microspheres containing exenatide, a salt thereof, or an analog thereof, microspheres with good characteristics may be obtained by adjusting the polyvinyl alcohol concentration and pH value in the outer aqueous phase such that the microspheres have high entrapment efficiency, uniform particle sizes, and minimum residue of the polyvinyl alcohol.

In a manufacturing process of the exenatide-containing microspheres of the present invention, the amount of cleaning solution, the freeze-drying time and temperature all play key roles in reducing the amount of acetic acid in the prepared microspheres.

The microspheres of the present invention may be washed with a cleaning solution, and the cleaning solution may be distilled water or a buffer solution. The ratio by weight of the microspheres to the cleaning solution may be 1:250 and above, preferably 1:330 and above. Washing the microspheres with a cleaning solution at such ratios provides a basis for obtaining exenatide-containing microspheres having minimum residue of acetic acid.

Using distill water or an alkaline buffer as a cleaning solution for washing microspheres can maintain the pH value of the cleaning solution within a stable range. The alkaline buffer may be citrate buffer, phosphate buffer, acetate buffer or phthalate buffer. The cleaning solution preferably is distilled water.

In the process of manufacturing exenatide-containing microspheres of the present invention, the freeze-drying process typically may consist of a pre-freezing process, a sublimation drying process, repeated freezing and sublimating processes, and re-drying process. The freeze-drying procedure is a drying method in which the solution freezes into a solid, and then the solid sublimates, without becoming a liquid, under low temperature and low pressure. In a freeze-drying process of the present invention, the re-drying temperature is controlled, while the other procedures may follow the conventional methods.

In the freeze-drying process described in the present invention, when the sublimation drying procedure is completed, the re-drying procedure may be used to further remove the residual liquid as much as possible. In the freeze-drying process described in the present invention, the re-drying temperature plays an important role in the content of acetic acid containing in the prepared microspheres. The re-drying temperature used in the present invention is lower than the glass-transition temperature of the mixture of exenatide acetate and PLGA (emulsified) so as to obtain a composition of exenatide-containing microspheres with good characteristics and meeting the quality standard.

Using the above-described processes to manufacture exenatide-containing microspheres of the present invention, the contents of acetic acid may be controlled to maintain the bioavailabilities of the compositions during long storage, thereby extending the shelf lives of the compositions.

Embodiments of the present invention use exenatide acetate as a starting material. By reducing acetic acid content to an extremely low level, the stabilities of the microspheres are improved, thereby extending the shelf lives of the compositions.

A manufacturing process of the invention achieves the control of acetic acid concentration in the microspheres by selecting a certain amount of a cleaning solution and selecting specific freeze-drying time and temperature. In the manufacturing process, the microspheres with good characteristics can be obtained by adjusting the polyvinyl alcohol concentration and the pH value in the outer aqueous phase, thereby achieving the characteristics of high entrapment efficiencies, uniform particle sizes, and minimum residue of the polyvinyl alcohol.

## DETAILED DESCRIPTION

The present invention will be further illustrated with the following examples, which should not limit the scope of the present invention in any way.

**Example 1: The microspheres of exenatide or a salt or an analogue thereof are prepared with an outer aqueous phase containing 0.5% polyvinyl alcohol (pH=6.5).**

Preparation of the polyvinyl alcohol solution (PVA solution): an appropriate amount of solid polyvinyl alcohol (average molecular weight of 13000-23000 Dalton) and a calculated volume of water were weighed. Then, the solid polyvinyl alcohol was dissolved in half of the water, and the other half of water was not added until the solid polyvinyl alcohol had dissolved completely. The PVA aqueous solution was stirred until mixed thoroughly, and then its pH value was measured. The pH value of the PVA aqueous solution was adjusted by adding 0.1mol/L hydrochloric acid solution or 0.1mol/L sodium hydroxide solution until it stabilized at 6.5.

Preparation method: a certain amount of exenatide specified in the formulation was weighed and dissolved in distilled water under stirring, so as to obtain a solution. A certain amount of copolymers of lactide and glycolide (PLGA) specified in the formulation was weighed and dissolved in dichloromethane ($CH_2Cl_2$) under stirring, so as to obtain a solution. The exenatide aqueous solution was added into the PLGA solution to obtain a primary emulsion. The prepared PVA aqueous solution was subjected to sterile filtration, then added into a vacuum emulsification blender (referred herein as microspheres preparation kettle) and cooled to 7-13 °C to obtain an outer aqueous phase. The primary emulsion was added into the microspheres preparation kettle under homogenization speed of 400 rpm, then it was homogeneously emulsified for 60s. Then, it was emulsified under a reduced homogenization speed of 150 rpm for 5 h to obtain solid microspheres, which were then filtered and collected. Then, the microspheres are rinsednwith water (see example 11 for the detailed method) to remove the PVA residue. The microspheres are then transferred into a freeze-drying tray, and mannitol and a proper amount of water for injection were then added therein. The freeze-drying tray was placed in a freeze drier for freeze-drying (see example 11 for the detailed method). The freeze-dried product was subjected to sieving and mixing to obtain the microspheres.

See tables 1-3 for results of assessments of entrapment efficiency, particle sizes and polymer residue.

**Example 2: The microspheres of exenatide or a salt or an analogue thereof are prepared in an outer aqueous phase of 0.8% polyvinyl alcohol aqueous solution (pH=5.1).**

Preparation of the polyvinyl alcohol aqueous solution (PVA aqueous solution): weight an appropriate amount of solid polyvinyl alcohol (average molecular weight of 13000-23000 Dalton) and prepare a calculated volume of water. Then, the solid polyvinyl alcohol was dissolved in half of the water, and the other half of water was not added until the solid polyvinyl alcohol had dissolved completely. The PVA aqueous solution was stirred and mixed thoroughly, and then its pH value was measured. The pH value of the PVA aqueous solution was adjusted with 0.1mol/L hydrochloric acid aqueous solution or 0.1mol/L sodium hydroxide aqueous solution until it stabilized at 5.1.

Preparation method: preparing the microspheres as described in example 1.

See tables 1-3 for results of assessments of entrapment efficiency, particle sizes and polymer residue.

**Example 3: The microspheres of exenatide or a salt or an analogue thereof are prepared in an outer aqueous phase of 1.0% polyvinyl alcohol aqueous solution (pH=5.6).**

Preparation of the polyvinyl alcohol aqueous solution (PVA aqueous solution): weigh an appropriate amount of solid polyvinyl alcohol (average molecular weight of 13000-23000 Dalton) and prepare a calculated volume of water. Then, the solid polyvinyl alcohol was dissolved in half of the water, and the other half of water was not added until the solid polyvinyl alcohol had dissolved completely. The PVA aqueous solution was stirred and mixed thoroughly, and then its pH value was measured. The pH value of the PVA aqueous solution was adjusted by adding phthalate buffer(pH=5.6) until it stabilized at 5.6.

Preparation method: preparing the microspheres as described in example 1.

See tables 1-3 for the results of assessments of entrapment efficiency, particle sizes and polymer residue.

**Example 4: The microspheres of exenatide or a salt or an analogue thereof are prepared in an outer aqueous phase of 1.2% polyvinyl alcohol aqueous solution (pH=6.1).**

Preparation of the polyvinyl alcohol aqueous solution (PVA aqueous solution): weigh an appropriate amount of solid polyvinyl alcohol (average molecular weight of 13000-23000 Dalton) and prepare a calculated volume of water. Then, the solid polyvinyl alcohol was dissolved in half of the water, and the other half of water was not added until the solid polyvinyl alcohol had dissolved completely. The PVA aqueous solution was stirred and mixed thoroughly, and then its pH value was measured. The pH value of the PVA aqueous solution was adjusted by adding 0.1mol/L hydrochloric acid aqueous solution or 0.1mol/L sodium hydroxide aqueous solution until it stabilized at 6.1.

Preparation method: preparing the microspheres as described in example 1.

See tables 1-3 for results of assessments of entrapment efficiency, particle sizes and polymer residue.

**Example 5: The microspheres of exenatide or a salt or an analogue thereof are prepared in an outer aqueous phase of 1.5% polyvinyl alcohol aqueous solution (pH=4.6).**

Preparation of the polyvinyl alcohol aqueous solution (PVA aqueous solution): weigh an appropriate amount of solid polyvinyl alcohol (average molecular weight of 13000-23000 Dalton) and prepare a calculated volume of water. Then, the solid polyvinyl alcohol was dissolved in half of the water, and the other half of water was not added until the solid polyvinyl alcohol had dissolved completely. The PVA aqueous solution was stirred and mixed thoroughly, and then its pH value was measured. The pH value of the PVA aqueous solution was adjusted by adding 0.1mol/L hydrochloric acid aqueous solution or 0.1mol/L sodium hydroxide aqueous solution until it stabilized at 4.6.
Preparation method: preparing the microspheres as described in example 1.
See tables 1-3 for results of assessments of entrapment efficiency, particle sizes and polymer residue.

**Example 6: The microspheres of exenatide or a salt or an analogue thereof are prepared in an outer aqueous phase of 1.0% polyvinyl alcohol aqueous solution (pH=6.5).**

Preparation of the polyvinyl alcohol aqueous solution (PVA aqueous solution): weigh an appropriate amount of solid polyvinyl alcohol (average molecular weight of 13000-23000 Dalton) and prepare a calculated volume of water. Then, the solid polyvinyl alcohol was dissolved in half of the water, and the other half of water was not added until the solid polyvinyl alcohol had dissolved completely. The PVA aqueous solution was stirred and mixed thoroughly, and then its pH value was measured. The pH value of the PVA aqueous solution was adjusted by adding 0.1mol/L hydrochloric acid aqueous solution or 0.1mol/L sodium hydroxide aqueous solution until it stabilized at 6.5.
Preparation method: preparing the microspheres as described in example 1.
See tables 1-3 for results of assessments of entrapment efficiency, particle sizes and polymer residue.

**Example 7: The microspheres of exenatide or a salt or an analogue thereof are prepared in an outer aqueous phase of 1.0% polyvinyl alcohol aqueous solution (pH=4.7).**

Preparation of the polyvinyl alcohol aqueous solution (PVA aqueous solution): weigh an appropriate amount of solid polyvinyl alcohol (average molecular weight of 13000-23000 Dalton) and prepare a calculated volume of water. Then, the solid polyvinyl alcohol was dissolved in half of the water, and the other half of water was not added until the solid polyvinyl alcohol had dissolved completely. The PVA aqueous solution was stirred and mixed thoroughly, and then its pH value was measured. The pH value of the PVA aqueous solution was adjusted by adding 0.1mol/L hydrochloric acid aqueous solution or 0.1mol/L sodium hydroxide aqueous solution until it stabilized at 4.7.
Preparation method: preparing the microspheres as described in example 1.
See tables 1-3 for results of assessments of entrapment efficiency, particle sizes and polymer residue.

**Example 8: The microspheres of exenatide or a salt or an analogue thereof are prepared in an outer aqueous phase of 1.0% polyvinyl alcohol aqueous solution (pH=5.6).**

Preparation of the polyvinyl alcohol aqueous solution (PVA aqueous solution): weigh an appropriate amount of solid polyvinyl alcohol (average molecular weight of 13000-23000 Dalton) and prepare a calculated volume of water. Then, the solid polyvinyl alcohol was dissolved in half of the water, and the other half of water was not added until the solid polyvinyl alcohol had dissolved completely. The PVA aqueous solution was stirred and mixed thoroughly, and then its pH value was measured. The pH value of the PVA aqueous solution was adjusted by adding phthalate buffer (pH=5.6) until it stabilized at 5.6.
Preparation method: preparing the microspheres as described in example 1.
See tables 1-3 for results of assessments of entrapment efficiency, particle sizes and polymer residue.

**Comparative example 1: The microspheres of exenatide or a salt or an analogue thereof are prepared in an outer aqueous phase of 1.0% polyvinyl alcohol aqueous solution (pH=9.0).**

Preparation of the polyvinyl alcohol aqueous solution (PVA aqueous solution): weigh an appropriate amount of solid polyvinyl alcohol (average molecular weight of 13000-23000 Dalton) and prepare a calculated volume of water. Then, the solid polyvinyl alcohol was dissolved in half of the water, and the other half of water was not added until the solid polyvinyl alcohol had dissolved completely. The PVA aqueous solution was stirred and mixed thoroughly, and then its pH value was measured. The pH value of the PVA aqueous solution was adjusted by adding 0.1mol/L hydrochloric acid aqueous solution or 0.1mol/L sodium hydroxide aqueous solution until it stabilized at 9.0.

Preparation method: preparing the microspheres as described in example 1.

See tables 1-3 for results of assessments of entrapment efficiency, particle sizes and polymer residue.

**Comparative example 2: The microspheres of exenatide or a salt or an analogue thereof are prepared in an outer aqueous phase of 1.0% polyvinyl alcohol aqueous solution (pH=3.0).**

Preparation of the polyvinyl alcohol aqueous solution (PVA aqueous solution): weigh an appropriate amount of solid polyvinyl alcohol (average molecular weight of 13000-23000 Dalton) and prepare a calculated volume of water. Then, the solid polyvinyl alcohol was dissolved in half of the water, and the other half of water was not added until the solid polyvinyl alcohol had dissolved completely. The PVA aqueous solution was stirred and mixed thoroughly, and then its pH value was measured. The pH value of the PVA aqueous solution could be adjusted by adding 0.1mol/L hydrochloric acid aqueous solution or 0.1mol/L sodium hydroxide aqueous solution until it stabilized at 3.0.

Preparation method: preparing the microspheres as described in example 1.

See tables 1-3 for results of assessments of entrapment efficiency, particle sizes and polymer residue.

**Comparative example 3: The microspheres of exenatide or a salt or an analogue thereof are prepared in an outer aqueous phase of 5.0% polyvinyl alcohol aqueous solution (pH=5.8).**

Preparation of the polyvinyl alcohol aqueous solution (PVA aqueous solution): weigh an appropriate amount of solid polyvinyl alcohol (average molecular weight of 13000-23000 Dalton) and prepare a calculated volume of water. Then, the solid polyvinyl alcohol was dissolved in half of the water, and the other half of water was not added until the solid polyvinyl alcohol had dissolved completely. The PVA aqueous solution was stirred and mixed thoroughly, and then its pH value was measured. The pH value of the PVA aqueous solution was adjusted by adding 0.1mol/L hydrochloric acid aqueous solution or 0.1mol/L sodium hydroxide aqueous solution until it stabilizesdat 5.8.

Preparation method: preparing the microspheres as described in example 1.

See tables 1-3 for results of assessments of entrapment efficiency, particle sizes and polymer residue.

**Comparative example 4: The microspheres of exenatide or a salt or an analogue thereof are prepared in an outer aqueous phase of 0.1% polyvinyl alcohol aqueous solution (pH=5.6).**

Preparation of the polyvinyl alcohol aqueous solution (PVA aqueous solution): weigh an appropriate amount of solid polyvinyl alcohol (average molecular weight of 13000-23000 Dalton) and prepare a calculated volume of water. Then, the solid polyvinyl alcohol was dissolved in half of the water, and the other half of water was not added until the solid polyvinyl alcohol had dissolved completely. The PVA aqueous solution was stirred and mixed thoroughly, and then its pH value was measured. The pH value of the PVA aqueous solution was adjusted by adding 0.1mol/L hydrochloric acid aqueous solution or 0.1mol/L sodium hydroxide aqueous solution until it stabilized at 5.6.

Preparation method: preparing the microspheres as described in example 1.

See tables 1-3 for results of assessments of entrapment efficiency, particle sizes and polymer residue.

**Test Example 1: measurement of entrapment efficiency for the microspheres of exenatide or a salt or an analogue thereof.**

The entrapment efficiency of the microspheres of exenatide or a salt or an analogue thereof may be determined as follows: 20 mg microspheres prepared in the examples were weighed and dissolved in 2 ml glacial acetic acid under ultrasoni-cation in a 10 ml volumetric flask to obtain a clear and transparent solution. The solution was diluted with water to volume, and then mixed and allowed to stand until the insoluble substance settled. Then, a supernatant was obtained and used as a test solution. An appropriate amount of reference exenatide was precisely weighed and dissolved in water, and then diluted to obtain a reference solution having a concentration of about 0.1mg/ml. A test solution ($20\mu$l) and a reference solution ($20\mu$l) were weighed, respectively. Then, both were injected into a liquid chromatograph. Record the chromatograms, and then determine the content of exenatide in the test solution using an external standard method based on the peak area.

The entrapment efficiency is expressed as % of the actual entrapment quantity relative to the theoretical quantity. See table 1 for the measurement results.

Table 1 Entrapment efficiency of the microspheres of exenatide or a salt or an analogue thereof

| Test NO. | Entrapment efficiency |
| --- | --- |
| Example 1 | 85.4% |
| Example 2 | 91.6% |
| Example 3 | 99.1% |
| Example 4 | 90.3% |
| Example 5 | 88.3% |
| Example 6 | 93.1% |
| Example 7 | 92.5% |
| Example 8 | 85.8% |
| Comparative example 1 | 64.2% |
| Comparative example 2 | 69.1% |
| Comparative example 3 | 68.2% |
| Comparative example 4 | 66.4% |

Analysis of results: for the microspheres of exenatide or a salt or an analogue thereof prepared in examples 1-8, the entrapment efficiencies of the microspheres were not affected by methods of adding exenatide and solution, but were affected by the concentrations and the pH values of the outer aqueous phases. The entrapment efficiencies of the microspheres were always above 85% when the concentrations of the outer aqueous phases and the pH values were under controlled. In the comparative examples, the entrapment efficiencies of the microspheres were always below 70% when the concentrations and the pH values of the outer aqueous phases were out of the controlled range.

**Test Example 2: measurement of the particle sizes and distributions for the microspheres of exenatide or a salt or an analogue thereof.**

Based on particle size and particle size distribution measurement methods [The third method in Appendix XIX E of Chinese Pharmacopoeia (second part, 2010 Edition)], 0.1% Tween 20 solution was used as a dispersing agent. About 120ml of Tween 20 solution was transferred into the sample dispersion device of a particle size analyzer (The Malvern Mastersizer 2000 Particle Size Analyzer), and the rotational speed controller was adjusted so that the stirring was at 2,100 rpm. The background of dispersing agent was measured first. An appropriate amount of exenatide-containing microspheres was added into the dispersing agent. After the sample was distributed evenly, the particle sizes D90, D50 and D10 were measured in parallel for three times and the average numbers were taken. The particle size distribution span used in the present invention is defined according to the Guidelines for Microcapsules, Microspheres and Liposome Preparations, Appendix XIXE of Chinese Pharmacopoeia (2010 Edition) as follows:

$$\text{Particle Size Distribution Span} = (D90\text{-}D10)/\,D50$$

The particle size distribution spans were calculated according to the above formula. See table 2 for the measurement results.

| Test NO. | Span |
| --- | --- |
| Example 1 | 0.76 |
| Example 2 | 0.74 |
| Example 3 | 0.24 |
| Example 4 | 0.78 |
| Example 5 | 0.64 |

(continued)

| Test NO. | Span |
|---|---|
| Example 6 | 0.44 |
| Example 7 | 0.41 |
| Example 8 | 0.79 |
| Comparative example 1 | 2.01 |
| Comparative example 2 | 2.29 |
| Comparative example 3 | 2.04 |
| Comparative example 4 | 2.07 |

Analysis of results: for the microspheres of exenatide or a salt or an analogue thereof prepared in Examples 1-8, the particle size distribution spans of the microspheres were not affected by the addition methods of the exenatide and the solutions, but were affected by the concentrations and the pH values of the outer aqueous phases. The particle size distribution spans of the microspheres were always below 0.8, when the concentrations of the outer aqueous phases and the pH values were under control. According to the measured particle size distribution span results, the sizes of microspheres product were uniform. In the comparative examples, the particle size distribution spans of the microspheres were above 2.0, when the concentrations and the pH values of the outer aqueous phases were out of the controlled range, indicating that the sizes of microspheres product were not uniform.

**Test Example 3: measurement of the content of polyvinyl alcohol residue in the microspheres of exenatide or a salt or an analogue thereof**

Determination method of the content of polyvinyl alcohol residue:

• The chromatographic conditions were as follows:

• Stationary phase: the gel chromatography column (TSK-GEL G2000SWXL)

• Mobile phase: the potassium dihydrogen phosphate buffer solution pH 7.0

• Flow rate: 0.8 ml/min.

• The differential refractive index detector was used.

Determination of a standard curve: an appropriate amount of PVA was precisely weighted and used to prepare a 1.0 mg/ml PVA mother solution. Then, the mother solution was diluted to prepare a series of standard solutions. The diluted concentrations were 0.01, 0.02, 0.05, 0.1, 0.2 and 0.3 mg/ml, respectively. Ten (100) μl of each standard solution was injected into the liquid chromatograph. Record the chromatograms, and then the standard curve was established by sample concentrations as the X-axis and peak areas as the Y-axis. The linear regression coefficient was not lower than 0.999 in the linear regression model.

Measurement method: 500 mg of microspheres of the present invention were precisely weighed and degraded in 50ml of 2 mol/L sodium hydroxide solution for 36 h. The solution was neutralized with 2 mol/L hydrochloric acid solution. The PLGA degradation products were removed by dialysis for 36 h in a dialysis bag having a cutoff of 3500 Dalton. Then, the solution was freeze-dried to obtain the polyvinyl alcohol residue. The polyvinyl alcohol residue was re-dissolved and diluted to 2 ml to obtain a test solution. Ten (100) μl test solution was injected into the liquid chromatograph. Then, record the chromatograms to calculate the contents of polyvinyl alcohol residues in microspheres using an external standard method.

Table 3 shows contents of polyvinyl alcohol residues in the microspheres of exenatide or a salt or an analogue thereof

| Test NO. | polyvinyl alcohol residue |
|---|---|
| Example 1 | 0.0067% |
| Example 2 | 0.0054% |

(continued)

| Test NO. | polyvinyl alcohol residue |
| --- | --- |
| Example 3 | 0.0019% |
| Example 4 | 0.0068% |
| Example 5 | 0.0079% |
| Example 6 | 0.0074% |
| Example 7 | 0.0043% |
| Example 8 | 0.0072% |
| Comparative example 1 | 0.0281% |
| Comparative example 2 | 0.0263% |
| Comparative example 3 | 0.0397% |
| Comparative example 4 | 0.0157% |

Analysis of results: for the microspheres of exenatide or a salt or an analogue thereof prepared in examples 1-8, the contents of polyvinyl alcohol residues in the microspheres were not affected by the concentrations and the pH values of the outer aqueous phases. The measured contents of polyvinyl alcohol residues in the microspheres prepared in examples 1-7 are below 0.008%. These microspheres can be safely used in the clinics because there are only minimum contents of polyvinyl alcohol residues in these microspheres. In the comparative examples, the contents of polyvinyl alcohol residues in the microspheres were above 0.01%, which are relatively high.

**Example 9: preparation of the exenatide-containing microspheres with a weight ratio of the (exenatide acetate + PLGA) to the cleaning solution of 1:250.**

Preparation method: 7.5 g exenatide acetate was weighed and dissolved in 25 ml distilled water under stirring to obtain a solution. Dissolve 142.5 g of a copolymer of lactide and glycolide (PLGA 5050 2A 16000) in 675 ml dichloromethane ($CH_2Cl_2$) under stirring to obtain another solution. Then, the exenatide acetate solution was mixed with the PLGA solution to obtain a primary emulsion. Subject 75L of the prepared 1%PVA aqueous solution (see example 3 for the detailed preparation method) to sterile filtration. It was then added into a vacuum emulsification blender (referred herein as microspheres preparation kettle) and cooled to 7-13°C for use as an outer aqueous phase. The primary emulsion was added into the microspheres preparation kettle and homogenized at rate of 400 rpm. Then, it was homogeneously emulsified for 60s, and then emulsified at a reduced homogenization rate of 150 rpm for 5 h to obtain solid microspheres, which were then filtered and collected. The microspheres were rinsed with 37.5Kg distilled water, and then transferred into a freeze-drying tray. An appropriate amount of mannitol injection solution and an appropriate amount of water were added into the freeze-drying tray, and then the freeze-drying tray was placed in a freeze drier to freeze-dry for 36 h at 40 °C (the glass-transition temperature of the mixture of exenatide acetate and PLGA is 43 °C). Then, the freeze-dried product was subjected to sieving and mixing to obtain the exenatide-containing microspheres.
See table 4 for results of assessments of the contents of acetic acid in the microspheres.

**Example 10: Preparation of the exenatide-containing microspheres with a weight ratio of the (exenatide acetate + PLGA) to the cleaning solution of 1:300.**

Preparation method: Weigh 7.5 g of exenatide acetate and dissolve it in 25 ml distilled water under stirring to obtain a solution. Dissolve 142.5 g of a copolymer of lactide and glycolide (PLGA 5050 2A 16000) in 675 ml of dichloromethane ($CH_2Cl_2$) under stirring to obtain another solution. Then, the exenatide acetate solution was mixed with the PLGA solution to obtain a primary emulsion. Subject 75L of the prepared 1%PVA aqueous solution (see example 3 for the detailed preparation method) to sterile filtration. Then, it was added into a vacuum emulsification blender (referred herein as microspheres preparation kettle) and cooled to 7-13 °C for use as an outer aqueous phase. The primary emulsion was added into the microspheres preparation kettle under a homogenization rate of 400 rpm. It was homogeneously emulsified for 60s, and then it was emulsified under a reduced homogenization rate of 150 rpm for 4h to obtain the solid microspheres, which were then filtered and collected. The microspheres were rinsed with 45Kg distilled water, and then transferred into a freeze-drying tray. An appropriate amount of mannitol injection solution and an appropriate amount of water were added into the freeze-drying tray. Then, the freeze-drying tray was placed in a freeze drier to free dry for 36 h at 40 °C (the glass-transition temperature of the mixture of exenatide acetate and PLGA is 43 °C). Then, the freeze-dried product was subjected to sieving and mixing to obtain the microspheres.

See table 4 for the determination and results of the contents of acetic acid in the microspheres.

**Example 11: Preparation of the exenatide-containing microspheres with a weight ratio of the (exenatide acetate + PLGA) to the cleaning solution of 1:330.**

Preparation method: Weigh 7.5 g of exenatide acetate and dissolve it in 25 ml of distilled water under stirring to obtain a solution. Dissolve 142.5 g of a copolymer of lactide and glycolide (PLGA 5050 2A 16000) in 675 ml of dichloromethane ($CH_2Cl_2$) under stirring to obtain another solution. Then, the exenatide acetate solution was mixed with the PLGA solution to obtain a primary emulsion. Subject 75 L of the prepared 1%PVA aqueous solution (see example 3 for the detailed preparation method) to sterile filtration. Then, it was added into a vacuum emulsification blender (referred herein as microspheres preparation kettle) and cooled to 7-13 °C for use as an outer aqueous phase. The primary emulsion was added into the microspheres preparation kettle under a homogenization rate of 400 rpm. It was homogeneously emulsified for 60s, and then it was emulsified under a reduced homogenization rate of 150 rpm for 4 h to obtain the solid microspheres, which were then filtered and collected. The microspheres were rinsed with 49.5 Kg of distilled water and then transferred into a freeze-drying tray. An appropriate amount of mannitol injection solution and an appropriate amount of water were added into the freeze-drying tray. Then, the freeze-drying tray was placed in a freeze drier to freeze dry for 36 h at 40 °C (the glass-transition temperature of the mixture of exenatide acetate and PLGA is 43 °C). Then, the freeze-dried product was subjected to sieving and mixing to obtain the microspheres.

See table 4 for the determination and results for the contents of acetic acid in the microspheres.

**Example 12: Preparation of the exenatide-containing microspheres with a weight ratio of the (exenatide acetate + PLGA) to the cleaning solution of 1:400.**

Preparation method: Weigh 7.5 g of exenatide acetate and dissolve it in 25 ml of distilled water under stirring to obtain a solution. Dissolve 142.5 g of a copolymer of lactide and glycolide (PLGA 5050 2A 16000) in 675 ml of dichloromethane ($CH_2Cl_2$) under stirring to obtain another solution. Then, the exenatide acetate solution was mixed with the PLGA solution to obtain a primary emulsion. Subject 75 L of the prepared 1%PVA aqueous solution (see example 3 for the detailed preparation method) to sterile filtration. Then, it was added into a vacuum emulsification blender (referred herein as microspheres preparation kettle) and cooled to 7-13 °C for use as an outer aqueous phase. The primary emulsion was added into the microspheres preparation kettle under a homogenization rate of 400 rpm. Then, it was homogeneously emulsified for 60s. Then, it was emulsified under a reduced homogenization rate of 150 rpm for 4h to obtain the solid microspheres, which were then filtered and collected. The microspheres were rinsed with 60Kg of distilled water, and then transferred into a freeze-drying tray. An appropriate amount of mannitol injection solution and an appropriate amount of water were added into the freeze-drying tray, and then the freeze-drying tray was placed in a freeze drier to freeze-dry for 36h at 40 °C (the glass-transition temperature of the mixture of exenatide acetate and PLGA is 43 °C). Then, the freeze-dried product was subjected to sieving and mixing to obtain the microspheres.

See table 4 for the determination and results for the content of acetic acid in the microspheres.

**Example 13: Preparation of the exenatide-containing microspheres using 38 °C as the re-drying temperature.**

Preparation method: Weigh 7.5 g of exenatide acetate and dissolve it in 25ml distilled water under stirring to obtain a solution. Dissolve 142.5g copolymers of lactide and glycolide (PLGA 5050 2A 16000) in 675 ml of dichloromethane ($CH_2Cl_2$) under stirring to obtain another solution. Then, the exenatide acetate solution was mixed with the PLGA solution to obtain a primary emulsion. Subject 75 L of the prepared 1%PVA aqueous solution (see example 3 for the detailed preparation method) to sterile filtration, then added into a vacuum emulsification blender (referred herein as microspheres preparation kettle) and cooled to 7-13 °C for use as an outer aqueous phase. The primary emulsion was added into the microspheres preparation kettle under a homogenization rate of 400 rpm. It was homogeneously emulsified for 60s, and then it was emulsified under a reduced homogenization rate of 150 rpm for 4h to obtain the solid microspheres, which were then filtered and collected. The microspheres were rinsed with 49.5 Kg of distilled water, and then transferred into a freeze-drying tray. An appropriate amount of mannitol injection solution and an appropriate amount of water were added into the freeze-drying tray, and then the freeze-drying tray was placed in a freeze drier to freeze-dry for 96 h at 38 °C (the glass-transition temperature of the mixture of exenatide acetate and PLGA is 43 °C). Then, the freeze-dried product was subjected to sieving and mixing to obtain the microspheres.

See table 4 for the determination and results for the content of acetic acid in the microspheres.

**Comparative example 5: Preparation of the exenatide-containing microspheres comprising more than 0.01 % acetic acid by weight.**

Weigh 7.5g exenatide acetate and dissolve it in 25 ml of distilled water under stirring to obtain a solution. Dissolve 142.5 g of a copolymer of lactide and glycolide (PLGA 5050 2A) in 675 ml of dichloromethane ($CH_2Cl_2$) under stirring to obtain another solution. Then, the exenatide acetate solution was mixed with the PLGA solution to obtain a primary emulsion. Subject 75L of the prepared 1% PVA aqueous solution (see example 3 for the detailed preparation method) to sterile filtration, then added into a vacuum emulsification blender (referred herein as microspheres preparation kettle) and cooled to 7-13 °C for use as an outer aqueous phase. The primary emulsion was added into the microspheres preparation kettle under a homogenization rate of 400 rpm. It was homogeneously emulsified for 60s, and then it was emulsified under a reduced homogenization rate of 150 rpm for 4h to obtain the solid microspheres, which were then filtered and collected. The microspheres were rinsed with 30Kg of distilled water, and then transferred into a freeze-drying tray. An appropriate amount of mannitol injection solution and an appropriate amount of water were added into the freeze-drying tray, and then the freeze-drying tray was placed in a freeze drier to freeze-dry for 36 h at 38 °C (the glass-transition temperature of the mixture of exenatide acetate and PLGA is 43 °C). Then, the freeze-dried product was subjected to sieving and mixing to obtain the microspheres.
See table 4 for the determination and results for the content of acetic acid in the microspheres.

**Test example 4: determination of the contents of acetic acid in different batches of exenatide-containing microspheres.**

Test method: determine with gas chromatography [Appendix VE, Method 3, of Chinese Pharmacopoeia (second part, 2000 Edition)].

**Chromatographic conditions and system suitability test**

The column was a 10 meters long capillary column with inner diameter of 0.32mm, and the inner layer was coated with 0.33μm of FFAP-CB fused silica. The chromatographic conditions were set as follows:

Injection temperature: 220 °C;
Detector temperature: 250 °C;
Split ratio: 100: 1;

The column temperature program was set as follows:

starting at temperature of 50 °C, and remained the same for 0.10 minutes;

(1) increasing the temperature at a rate of 30 °C / min;
(2) the final temperature of 230 °C, and remained for 5 minutes;
(3) Injection volume: 1μl;

The number of theoretical plates: calculated according to acetate peak and should not be less than 5000. The resolution of acetic acid peak and the internal standard peak should conform to the specifications.
**Determination of the correction factor:** 1.0 ml of n-hexadecane was precisely weighed and dissolved in 30 ml of dimethylformamide in a 50 ml volumetric flask, diluted to volume and shaken well to be used as an internal standard solution. 625 mg of acetic acid reference was precisely weighed and dissolved in dimethylformamide in a 100 ml volumetric flask, diluted to volume and shaken well before use. Ten (10) ml of the aforesaid solution was transferred into a 100 ml volumetric flask. Five (5) ml of the internal standard solution was added. The solution was dissolved with dimethylformamide and diluted to volume and shaken well. One (1)μl of the solution was injected into gas chromatography and injected continually for 3-5 times. The correction factor was calculated according to average peak areas.
**Test sample preparation and measurement:** About 50 mg of goserelin-containing microspheres prepared according to Example 1 was weighed and transferred into a 2 ml volumetric flask, into which 1 ml of dimethylformamide was added to dissolve the sample. One hundred (100) μl internal standard solution was added precisely and then the flask was brought to final volume with dimethylformamide and shaken well. One (1) μl sample was injected into gas chromatograph. The result was calculated by an internal standard method. Results are shown in Table 4.

Table 4 Determination of the content of acetic acid in exenatide-containing microspheres.

| Test NO. | Content of acetic acid |
| --- | --- |
| Example 9 | 0.0094% |
| Example 10 | 0.0036% |
| Example 11 | 0.0017% |
| Example 12 | 0.0021% |
| Example 13 | 0.0045% |
| Comparative example 5 | 0.0732% |

Analysis of results: for the microspheres of exenatide or a salt or an analogue thereof prepared in examples 9-13, the microspheres were rinsed with a cleaning solution, and the ratio by weight of the (exenatide acetate + PLGA) to the cleaning solution is 1:250. The re-drying temperature was set lower than the glass-transition temperature of the mixture of exenatide acetate and PLGA, and the contents of acetic acid in the microspheres were always below 0.01%. The same method was used in the comparative examples, the amount of the cleaning solution is lower than 250 times than the amount of (exenatide acetate + PLGA), and the contents of acetic acid in the microspheres were always above 0.01%.

**Test Example 5: comparative in vivo release experiment of the exenatide-containing microspheres before and after the stability test**

Test materials:

Test drugs: the exenatide-containing microspheres in Comparative Example 5, Examples 11 and 13 were studied for stability by conducting in vivo release test between samples immediately after preparation (0 month) and after storage for 6 months at temperature of 40°C and humidity of 75%. The test results are showed in Table 5.

Experimental animals:

- SD rats (Shandong Luye Pharmaceutical Co., Ltd. Animal Room).
- Test instruments:
- a QTRAP5500 mass spectrometer fitted with an ion-spray ionization source (Applied Biosystem, Inc.);
- an Agilent 1290 high performance liquid chromatography system comprising a dual infusion pump, an autosampler and a column oven;
- an Anke TGL-16G Feige desk centrifuge, (Shanghai Anting Scientific Instrument Factory); and
- a Turbo Vap LV pressure blowing concentrator, (Biotage, Inc).

Test method:

Experimental animals: male SD rats with body weight of 240±20g, 16 per group;

- Route of administration and dose: abdominal subcutaneous injection at a dose of 2 mg/kg.
- Blood sampling time: blood samples were collected before (at 0h) and after administration at 1h, 6h, 1d, 2d, 3d, 5d, 7d, 9d, 11d, 14d, 16d, 18d, 21d, 23d, 25d, and 28d, respectively.
- Determination of biological samples: Enzyme linked immunosorbent assays method.
- Results are shown in Table 5.

Table 5 Blood concentrations of exenatide at different time after intramuscular injection to rats (ng/mL)

| Time (Day) | Comparative example 5 (0 months) | Comparative example 5 (40°C/75%, 6 months) | Example 11 (0 months) | Example 11 (40°C/75%, 6 months) | Example 13 (0 months) | Example 13 (40°C/75%, 6 months) |
|---|---|---|---|---|---|---|
| 0.042 | 1.16 | 0.74 | 1.16 | 1.25 | 1.07 | 1.05 |
| 0.25 | 0.26 | 0.17 | 0.42 | 0.39 | 0.57 | 0.67 |
| 1 | 0.24 | 0.29 | 0.13 | 0.27 | 0.46 | 0.29 |
| 2 | 0.76 | 1.48 | 1.69 | 0.29 | 0.97 | 1.81 |
| 3 | 1.31 | 3.14 | 1.47 | 2.58 | 4.37 | 3.67 |
| 5 | 3.3 | 3.89 | 5.54 | 4.47 | 4.26 | 4.67 |
| 7 | 6.13 | 3.41 | 9.54 | 9.98 | 10.59 | 9.69 |
| 9 | 8.93 | 5.76 | 5.91 | 6.67 | 4.26 | 5.22 |
| 11 | 4.11 | 1.87 | 2.57 | 1.67 | 1.89 | 1.67 |
| 14 | 1.17 | 1.09 | 1.06 | 1.27 | 1.29 | 1.05 |
| 16 | 0.92 | 0.87 | 0.99 | 0.94 | 0.87 | 0.77 |
| 18 | 0.48 | 0.19 | 0.38 | 0.63 | 0.56 | 0.76 |
| 21 | 0.31 | 0.09 | 0.21 | 0.47 | 0.21 | 0.34 |
| 23 | 0.1 | 0.14 | 0.19 | 0.11 | 0.31 | 0.47 |
| 25 | 0.11 | 0.1 | 0.07 | 0.04 | 0.04 | 0.07 |
| 28 | 0.05 | 0.02 | 0.04 | 0.01 | 0.02 | 0.03 |
| AUC(ng/ml*h) | 1379.75 | 1035.31 | 1427.52 | 1423.07 | 1414.07 | 1426.35 |

The results show that the contents of acetic acid during the manufacturing process of exenatide acetate microspheres have great effects on the bioavailabilities of the products within the storage periods. After the stability test (stored for 6 months at temperature of 40°C and humidity of 75%), the amounts of in vivo releases for the products did not change when the contents of acetic acid were below 0.01%. The bioavailabilities of the microspheres without control of the contents of acetic acid have declined by more than 20%.

**Industrial application**

The present invention provides exenatide-containing compositions, and the compositions ares in the form of microspheres. The exenatide-containing microspheres comprise exenatide acetate and a copolymer of lactide and glycolide (PLGA) as raw materials, and the contents of acetic acid in the prepared exenatide-containing composition are lower than 0.01%.

In the manufacturing process of the microspheres, an appropriate amount of applicable cleaning solution is used, an appropriate re-drying temperature is selected during the freeze-dried process, so as to obtain a composition of exenatide-containing microspheres with good characteristics meeting the quality standard, thereby improving the stability and extending the product shelf lives. In the manufacturing process of the microspheres, an appropriate outer aqueous phase is used to obtain microspheres with uniform particle size and minimum residue of the polymers, so as to provide a composition with better quality for the clinical use. The present invention is suitable for industrial applications.

**Claims**

1. An exenatide-containing composition, wherein the composition is in a form of microspheres and is prepared with exenatide acetate or a salt or an analogue thereof and a copolymer of lactide and glycolide (PLGA) as raw materials, wherein a content of acetic acid in the exenatide-containing composition is lower than 0.01%.

2. The composition according to claim 1, wherein a content of exenatide is 2-10% by weight, preferably 3-8%, and more preferably 4-6%.

3. The composition according to claim 2, wherein a content of the copolymer of lactide and glycolide (PLGA) is 90-98% by weight, preferably 92-97%, and more preferably 94-96%.

4. The composition according to claim 3, wherein a weight average molecular weight of the PLGA is 6,000-45,000 Dalton, preferably 10,000-30,000 Dalton, and more preferably 10,000-25,000 Dalton.

5. The composition according to claim 3, wherein a molar ratio of lactide to glycolide in the PLGA is from 90:10 to 10:90, preferably from 75:25 to 25:75; more preferably from 60:40 to 40:60, especially 50:50.

6. The composition according to claim 4, wherein the PLGA has an intrinsic viscosity of 0.10-0.40 dl/g, preferably 0.10-0.35 dl/g, more preferably 0.10-0.30 dl/g.

7. The composition according to claim 1-6, wherein the microspheres are prepared by emulsion-solvent evaporation technique, which includes adding the PLGA into an organic solvent to obtain an organic solution, then adding exenatide acetate into the prepared organic solution to obtain a primary emulsion, then adding the primary emulsion into an outer aqueous phase to form an emulsion, then solidifying and volatilizing the emulsion to remove the organic solvent; and then washing the residue, followed by freeze-drying the residue to obtain the microspheres.

8. The composition according to claim 7, wherein the microspheres are washed with a cleaning solution and then re-dried, wherein a weight ratio of microspheres to the cleaning solution should be appropriate, and the microspheres are re-dried at a selected temperature.

9. The composition according to claim 8, wherein the organic solvent can dissolve PLGA and is selected from dichloromethane, acetone and acetonitrile, preferably dichloromethane, wherein the cleaning solution is distilled water or alkaline buffer, preferably distilled water, and a weight ratio of microspheres to the cleaning solution is 1:250 and above, preferably above 1:330 and above.

10. The composition according to claims 8 or 9, wherein the re-drying temperature used in the freeze-drying process is lower than the glass-transition temperature of the mixture of exenatide acetate and PLGA.

11. The composition according to claim 7, wherein the outer aqueous phase is a polyvinyl alcohol solution, wherein a content of the polyvinyl alcohol in the composition is not more than 0.008% by weight.

12. The composition according to claim 11, wherein the content (by weight/volume %; (g/100ml)) of the polyvinyl alcohol in the outer aqueous phase is 0.5-1.5 w/v %, preferably 1 w/v %; wherein a pH value of the outer aqueous phase is $5.6 \pm 1.0$, preferably $5.6 \pm 0.5$, more preferably $5.6 \pm 0.2$.

13. The composition according to claim 12, wherein the pH value of the outer aqueous phase is adjusted with a water-soluble acid, base or buffer solution, wherein the acid solution is 0.1mol/L hydrochloric acid aqueous solution, the base solution is 0.1mol/L sodium hydroxide aqueous solution, the buffer solution is a phthalate buffer (pH=5.6).

14. The composition according to claims 11, 12 or 13, wherein a weight average molecular weight of the polyvinyl alcohol is 13,000-23,000 Dalton.

15. A method for preparing a composition of exenatide-containing microspheres, comprising using exenatide or a salt or an analogue thereof and a copolymer of lactide and glycolide (PLGA) as raw materials, and preparing the microspheres using emulsion-solvent evaporation technique, which comprises: adding the PLGA into an organic solvent to obtain an organic solution, then adding exenatide acetate into the prepared organic solution to obtain a primary emulsion, then adding the primary emulsion into an outer aqueous phase to form an emulsion, then solidifying and evaporating the emulsion to remove the organic solvent; and then washing and freeze-drying the residue to obtain freeze-dried microspheres.

16. The method according to claim 15, wherein the washing is with a cleaning solution at a selected ratio, and further comprising re-drying at a selected temperature.

**17.** The method according to claim 16, wherein the organic solvent can dissolve PLGA, and the organic solvent is selected from dichloromethane, acetone and acetonitrile, preferably dichloromethane; wherein the cleaning solution is distilled water or alkaline buffer solution, preferably distilled water, and a weight ratio of microspheres to the cleaning solution is 1:250 and above, preferably 1:330 and above.

**18.** The method according to claims 16 or 17, wherein the re-drying temperature used in the freeze-dried process is lower than the glass-transition temperature of the mixture of exenatide acetate and PLGA.

**19.** The method according to claim 15, wherein the outer aqueous phase is a polyvinyl alcohol solution, a content (by weight/volume; (g/100ml)) of the polyvinyl alcohol in the outer aqueous phase is 0.5-1.5 w/v %, preferably 1 w/v %; wherein a pH value of the solution is $5.6\pm1.0$, preferably $5.6\pm0.5$, more preferably $5.6\pm0.2$; and wherein a content of the polyvinyl alcohol in the composition is not more than 0.008% by weight.

**20.** The method according to claim 19, wherein the pH value of the outer aqueous phase is adjusted with a water-soluble acid, base or buffer solution, wherein the acid solution is 0.1mol/L hydrochloric acid aqueous solution, the base solution is preferably 0.1mol/L of sodium hydroxide aqueous solution, the buffer solution is phthalate buffer (pH=5.6).

# INTERNATIONAL SEARCH REPORT

| | International application No. |
| --- | --- |
| | **PCT/CN2015/076688** |

## A. CLASSIFICATION OF SUBJECT MATTER

A61K 9/16 (2006.01) i; A61K 38/22 (2006.01) i; A61K 47/34 (2006.01) i; A61P 3/10 (2006.01) i

According to International Patent Classification (IPC) or to both national classification and IPC

## B. FIELDS SEARCHED

Minimum documentation searched (classification system followed by classification symbols)

A61K A61P

Documentation searched other than minimum documentation to the extent that such documents are included in the fields searched

Electronic data base consulted during the international search (name of data base and, where practicable, search terms used)

WPI, EPODOC, CNPAT, CNKI, CA, MEDLINE, EMBASE: SHANDONG LUYE PHARMACEUTICAL, poly(lactide-co-glycolide, polylactic glycolic acid, Exenatide, microballoons, microsphere, PLGA, Diglycolide dilactide copolymer

## C. DOCUMENTS CONSIDERED TO BE RELEVANT

| Category* | Citation of document, with indication, where appropriate, of the relevant passages | Relevant to claim No. |
| --- | --- | --- |
| X | CN 103142475 A (SHENZHEN JYMED TECHNOLOGY CO., LTD.), 12 June 2013 (12.06.2013), claims 1-9, and embodiment 1 | 15-16 |
| A | CN 103142475 A (SHENZHEN JYMED TECHNOLOGY CO., LTD.), 12 June 2013 (12.06.2013), claims 1-9, and embodiment 1 | 1-14, 17-20 |
| X | CN 102198103 A (SHENZHEN HANYU PHARMACEUTICAL CO., LTD.), 28 September 2011 (28.09.2011), claims 1-10, and embodiment 1 | 15-16 |
| A | CN 102198103 A (SHENZHEN HANYU PHARMACEUTICAL CO., LTD.), 28 September 2011 (28.09.2011), claims 1-10, and embodiment 1 | 1-14, 17-20 |
| PX | CN 103932992 A (SHANDONG LUYE PHARMACEUTICAL CO., LTD.), 23 July 2014 (23.07.2014), claims 1-10, and description, paragraph 0091 | 1-10, 15-18 |
| PX | CN 103932992 A (SHANDONG LUYE PHARMACEUTICAL CO., LTD.), 23 July 2014 (23.07.2014), claims 1-10, and description, paragraph 0091 | 11-14 |
| PX | CN 103932993 A (SHANDONG LUYE PHARMACEUTICAL CO., LTD.), 23 July 2014 (23.07.2014), claims 1-10 | 15-16, 19-20 |

☒ Further documents are listed in the continuation of Box C.    ☒ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| 20 June 2015 (20.06.2015) | **22 July 2015 (22.07.2015)** |

| Name and mailing address of the ISA/CN: State Intellectual Property Office of the P. R. China No. 6, Xitucheng Road, Jimenqiao Haidian District, Beijing 100088, China Facsimile No.: (86-10) 62019451 | Authorized officer **YAO, Zhanghuan** Telephone No.: (86-10) **62411131** |
| --- | --- |

Form PCT/ISA/210 (second sheet) (July 2009)

## INTERNATIONAL SEARCH REPORT

International application No.

**PCT/CN2015/076688**

**C (Continuation).** DOCUMENTS CONSIDERED TO BE RELEVANT

| Category* | Citation of document, with indication, where appropriate, of the relevant passages | Relevant to claim No. |
|---|---|---|
| PY | CN 103932993 A (SHANDONG LUYE PHARMACEUTICAL CO., LTD.), 23 July 2014 (23.07.2014), claims 1-10 | 11-14 |

Form PCT/ISA/210 (continuation of second sheet) (July 2009)

## INTERNATIONAL SEARCH REPORT
Information on patent family members

International application No.

**PCT/CN2015/076688**

| Patent Documents referred in the Report | Publication Date | Patent Family | Publication Date |
|---|---|---|---|
| CN 103142475 A | 12 June 2013 | CN 103142475 B | 06 August 2014 |
| CN 102198103 A | 28 September 2011 | CN 102198103 B | 23 July 2014 |
| CN 103932992 A | 23 July 2014 | None | |
| CN 103932993 A | 23 July 2014 | None | |

Form PCT/ISA/210 (patent family annex) (July 2009)

**REFERENCES CITED IN THE DESCRIPTION**

**Non-patent literature cited in the description**

- Appendix XIX E of Chinese Pharmacopoeia. 2010
- Guidelines for Microcapsules, Microspheres and Liposome Preparations, Appendix XIXE of Chinese Pharmacopoeia. 2010
- Appendix VE, Method 3, of Chinese Pharmacopoeia. 2000